# EUROPEAN PATENT SPECIFICATION

(11) **EP 3 246 315 B1**
(45) Date of publication and mention of the grant of the patent: **25.08.2021**
(21) Application number: 16737040.2
(22) Date of filing: 06.01.2016
(51) Int. Cl.: C07D 223/10, C07D 201/04

(54) **CAPROLACTAM PREPARATION METHOD**
VERFAHREN ZUR HERSTELLUNG VON CAPROLACTAM
PROCÉDÉ DE PRÉPARATION DE CAPROLACTAME

(30) Priority: 15.01.2015 CN 201510019581
(43) Date of publication of application: 22.11.2017
(73) Proprietor: Hubei Jinxiangning Chemical Engineering Techenology Co., Ltd, Wuhan, Hubei 430075 (CN); Hubei Sanning Chemi-Industry Co., Ltd, Zhijiang, Hubei 443206 (CN)
(72) Inventor: LUO, Hean, Wuhan Hubei 430075 (CN); ZHAO, Chengjun, Wuhan Hubei 430075 (CN); REN, Chengyun, Wuhan Hubei 430075 (CN); WU, Jian, Wuhan Hubei 430075 (CN); LI, Wanqing, Zhijiang Hubei 443206 (CN); WEI, Tianrong, Wuhan Hubei 430075 (CN); LEI, Fengsheng, Wuhan Hubei 430075 (CN); CHEN, Hao, Wuhan Hubei 430075 (CN); ZHAO, Yun, Wuhan Hubei 430075 (CN)
(74) Representative: Nederlandsch Octrooibureau
(86) International application number: PCT/CN2016/070245
(87) International publication number: WO 2016/112814

(56) References cited:
- EP-A1- 0 735 017
- EP-A2- 0 208 311
- CN-A- 1 371 905
- CN-A- 101 168 524
- JP-A- 2000 080 067
- K. T. ZUIDHOF ET AL: "Beckmann rearrangement of cyclohexanone oxime to [epsilon]-caprolactam in microreactors", AI CH E JOURNAL, 1 January 2009 (2009-01-01), pages NA-NA, XP055487833, US ISSN: 0001-1541, DOI: 10.1002/aic.12051
- SONG ZHAO ET AL: "An investigation into cyclohexanone ammoximation over Ti-MWW in a continuous slurry reactor", APPLIED CATALYSIS A: GENERAL, ELSEVIER, AMSTERDAM, NL, vol. 394, no. 1, 17 October 2010 (2010-10-17), pages 1-8, XP028363544, ISSN: 0926-860X, DOI: 10.1016/J.APCATA.2010.10.037 [retrieved on 2010-12-30]

## Description

### Field of the Invention

The present invention relates to a caprolactam preparation method.

### Background of the Invention

Caprolactam is a raw material for producing nylon 6. At present, the caprolactam preparation methods in the world mainly include cyclohexanone-hydroxylamine method which utilizes benzene as the raw material, hexahydrobenzoic acid amidation method which utilizes methyl benzene as the raw material, and cyclohexane photonitrozation method, wherein, 98 % caprolactam is prepared with the cyclohexanone-hydroxylamine method.

K. T. Zuidhof et al., AIChE Journal, 2010, 56(5), pp 1297-1304, disclose Beckmann rearrangement of cyclohexanone oxime to ε-caprolactam in microreactors. The publication by S. Zhao et al., Applied Catalysis A: General, 2011, 394, pp 1-8, concerns an investigation into cyclohexanone ammoximation over Ti-MWW in a continuous slurry reactor.

EP0208311A2 discloses a catalytic process for preparing cyclohexanone-oxime. JP2000 -080067 discloses a method for producing an oxime in the presence of a titanosilicate catalyst. CN1371905A discloses a method for synthesizing ε-caprolactam. EP0735017A1 discloses an integrated process for cyclohexanone oxime production. CN101168524A discloses a method for preparing low sulfonated caprolactam.

The process of preparing caprolactam with the existing cyclohexanone-hydroxylamine method usually comprises: preparing cyclohexanone oxime from cyclohexanone through an ammoximation reaction, preparing caprolactam sulfonate from cyclohexanone oxime through a Beckmann rearrangement reaction, and preparing caprolactam from caprolactam sulfonate through a neutralization reaction. In the process of preparing caprolactam with the cyclohexanone-hydroxylamine method, preparing cyclohexanone oxime through ammoximation in one step is an important improvement in 70 years in industrial production of caprolactam. It not only shortens the caprolactam preparation process and reduces the demanding requirements for conditions and raw materials, but also simplifies the operation, reduces the ammonium sulfate byproduct, and decreases the production cost.

When cyclohexanone oxime is produced through the existing ammoximation process, an inert organic substance that has a low boiling point and is flammable and explosive is usually used as a reacting solvent in the reaction system; consequently, the temperature of the ammoximation reaction should not be too high, and usually is only 60-84 °C; thus, on one hand, it is impossible to achieve high cyclohexanone conversion ratio and high selectivity for cyclohexanone oxime; on the other hand, the obtained product from ammoximation reaction usually has to be refined before it can be used in the follow-up Beckmann rearrangement reaction. Consequently, the equipment investment and energy consumption required for the follow-up separation and purification of the cyclohexanone oxime are increased. The steam consumption in the cyclohexanone oxime refinement procedure accounts for about 50 % of the steam consumption in the caprolactam production process. Moreover, when caprolactam is prepared with the existing method, the relative ratio of oleum to cyclohexanone oxime has to be increased properly in the procedure of Beckmann rearrangement reaction owing to the high viscosity of the reaction system, in order to ensure complete reaction of cyclohexanone oxime; as a result, the amount of the byproduct ammonium hydrogen sulfate is increased inevitably.

In summary, it is still an urgent task to improve the yield of caprolactam, shorten the process flow, and reduce the energy consumption in caprolactam production.

### Contents of the Invention

The object of the present invention is to provide an innovative caprolactam preparation method, so as to overcome the defects of low yield of caprolactam, high percentage of the byproduct ammonium sulfate, long process flow, and high energy consumption in caprolactam production with the existing caprolactam preparation method in the prior art.

Through in-depth research on the catalytic reaction mechanism of the ammoximation reaction in the caprolactam preparation process, the inventor has found: in the conventional ammoximation reaction, usually an organic solvent is used as the reaction medium, and the purpose of adding the organic solvent is to avoid clogging of the pore canals of the oximation catalyst by the resultant cyclohexanone oxime, which may cause degraded activity of the oximation catalyst. However, with such a solution, the reaction temperature is limited to a relative low temperature, and the cyclohexanone conversion ratio and the selectivity for cyclohexanone oxime are low.

The inventor abandons the conventional concept, directly uses pure water as a reaction medium for the ammoximation reaction, without adding any organic solvent, and increases the reaction temperature appropriately (within 80-100 °C range); thus, higher cyclohexanone conversion ratio and higher selectivity for cyclohexanone oxime are achieved, and thereby a higher caprolactam yield is obtained. The reason for that result may be: when water is used as the reaction medium, the ammoximation reaction temperature can be increased appropriately, and the increased reaction temperature leads to decreased viscosity of cyclohexanone oxime and improved fluidity of the liquid; the cyclohexanone oxime can escape from the pore canals of the oximation catalyst quickly, and the cyclohexanone oxime separation result is better; in addition, the potential activity of the oximation catalyst is activated at the high temperature.

Through in-depth research, the inventor has further found: if the product of the ammoximation reaction is directly extracted with an inert organic solvent, the cyclohexanone oxime can enter into the extract phase essentially; then, the extract phase that contains cyclohexanone oxime can be directly used in the Beckmann rearrangement reaction; thus, not only the cyclohexanone oxime refinement procedure is omitted and the process flow is shortened, but also the energy consumption and the amount of waste water are reduced. For a caprolactam installation with 100,000t/a capacity, energy consumption equivalent to a value of 40-50 million RMB can be reduced annually, and about 8,000 tons of waste water can be reduced annually; moreover, since an inert organic solvent is used, the viscosity of the rearrangement liquid can be decreased, the mass transfer effect can be improved, and thereby the quality of caprolactam can be increased, while the amount of oleum and the amount of the byproduct ammonium hydrogen sulfate can be reduced; specifically, the amount of oleum can be reduced by about 30%, and the amount of the byproduct ammonium hydrogen sulfate can be reduced by about 30%. In addition, since the Beckmann rearrangement reaction is an exothermic reaction, the inertial organic solvent in the Beckmann rearrangement reaction system can be recovered utilizing the heat generated in the rearrangement reaction, and can be reused. Therefore, the method is of high value for industrial application.

The inventor has obtained the present invention on the basis of the above-mentioned research findings.

Specifically, the caprolactam preparation method provided in the present invention comprises the following steps:
(1) Controlling cyclohexanone, ammonia, and hydrogen peroxide to have an ammoximation reaction in an aqueous solvent at 80-100 °C temperature in the presence of an oximation catalyst, without any organic solvent, to obtain a solution that contains cyclohexanone oxime;
(2) Extracting the obtained solution that contains cyclohexanone oxime in step (1) with an inert organic solvent, to obtain an extract phase that contains cyclohexanone oxime and a raffinate phase that contains the oximation catalyst and water;
(3) Controlling the extract phase to have a Beckmann rearrangement reaction with oleum, and controlling the product of the Beckmann rearrangement reaction to have a neutralization reaction with ammonia,
wherein step (2) further comprises: separating the oximation catalyst from the raffinate phase, and reusing the oximation catalyst in the ammoximation reaction, and
wherein step (3) further comprises: curing the product of the Beckmann rearrangement reaction after the Beckmann rearrangement reaction is finished and before the neutralization reaction is started, and then controlling the cured product to have the neutralization reaction; wherein the curing conditions are 100-140 °C curing temperature and 0.5-3 h curing time.

When caprolactam is prepared with the method provided in the present invention, the yield of caprolactam can be improved. Furthermore, with the method provided in the present invention, the refinement procedures such as water scrubbing and rectification, etc. in the cyclohexanone oxime preparation process can be omitted, the process flow and production cycle can be greatly shortened; moreover, the inert organic solvent can be recovered utilizing the heat generated in the rearrangement reaction and then reused. Thus, the annual yield is improved on the basis of the existing installation. With the method, great social and economic benefits can be obtained. Other features and advantages of the present invention will be further detailed in the embodiments hereunder.

### Detailed Description of the Embodiments

Hereunder some embodiments of the present invention will be detailed.

The caprolactam preparation method provided in the present invention comprises the following steps:
(1) Controlling cyclohexanone, ammonia, and hydrogen peroxide to have an ammoximation reaction in an aqueous solvent at 80-100 °C temperature in the presence of an oximation catalyst, without any organic solvent, to obtain a solution that contains cyclohexanone oxime;
(2) Extracting the obtained solution that contains cyclohexanone oxime in step (1) with an inert organic solvent, to obtain an extract phase that contains cyclohexanone oxime and a raffinate phase that contains the oximation catalyst and water;
(3) Controlling the extract phase to have a Beckmann rearrangement reaction with oleum, and controlling the product of the Beckmann rearrangement reaction to have a neutralization reaction with ammonia,
wherein step (2) further comprises: separating the oximation catalyst from the raffinate phase, and reusing the oximation catalyst in the ammoximation reaction, and
wherein step (3) further comprises: curing the product of the Beckmann rearrangement reaction after the Beckmann rearrangement reaction is finished and before the neutralization reaction is started, and then controlling the cured product to have the neutralization reaction; wherein the curing conditions are 100-140 °C curing temperature and 0.5-3 h curing time.

According to the present invention, preferably the aqueous solvent mainly contains water; alternatively, other inert inorganic solvents can be added as required. The aqueous solvent used in the embodiments of the present invention preferably is water.

There is no particular restriction on the amount of water in the ammoximation reaction system in the present invention. Namely, water may be added in an appropriate amount according to the actual amount of the raw material cyclohexanone for the reaction. Usually, with respect to 1 mol cyclohexanone, the amount of water in the ammoximation reaction system may be 6-500 mol. In addition, to obtain a higher cyclohexanone conversion ratio and higher selectivity for cyclohexanone oxime, decrease the difficulties in the follow-up separation and the amount of waste water, and improve the reaction efficiency, preferably, with respect to 1 mol cyclohexanone, the amount of water in the ammoximation reaction system is 20-50 mol. In the present invention, an appropriate amount of water may be used, as long as the above requirement is met. The water may be water carried by the raw material, or can be added additionally so that the above requirement is met; wherein, the amount of water refers to the total amount of water carried by the raw material and water added additionally.

If the temperature of the ammoximation reaction is controlled in the preferred range described above, the reaction is easier, and thereby the cyclohexanone conversion ratio and the selectivity for cyclohexanone oxime can be improved more remarkably. In addition, the pressure of the ammoximation reaction may be 50-300 kPa, preferably 200-300 kPa. In the present invention, the pressure refers to absolute pressure. Longer ammoximation reaction time is helpful for improving the conversion ratio and yield of the reaction product. However, the conversion ratio and yield of the reaction product will not be improved further apparently if the reaction time is excessively long. Therefore, in comprehensive consideration, the reaction time of the ammoximation reaction preferably is 10-300 min., more preferably is 50-100 min.

The hydrogen peroxide may be added in a form of hydrogen peroxide gas or hydrogen peroxide solution, preferably is added in a form of hydrogen peroxide solution; thus, the operation will be easier, and the mix ratio of the materials can be adjusted accurately. There is no particular restriction on the concentration of the hydrogen peroxide solution in the present invention; namely, the concentration may be selected reasonably according to the actual circumstance. For example, the hydrogen peroxide solution may be commercial hydrogen peroxide solution at 27.5 wt% concentration, 50 wt% concentration or 70 wt% concentration.

In the present invention, there is no particular restriction on the amounts of the materials used in the ammoximation reaction process in the step (1); namely, the amounts of the materials may be selected conventionally in the art. For example, in step (1), with respect to 1 mol cyclohexanone, the amount of the hydrogen peroxide may be 1-1.5 mol, preferably 1-1.25 mol; the amount of the ammonia may be 1-1.5 mol, preferably 1-1.25 mol.

The kind of the oximation catalyst may be selected conventionally in the art. For example, the oximation catalyst may generally be a titanium silicalite molecular sieve catalyst, preferably at least one of titanium silicalite molecular sieve that has a MFI structure (e.g., TS-1), titanium silicalite molecular sieve that has a MEL structure (e.g., TS-2), and titanium silicalite molecular sieve that has a BETA structure (e.g., T1-β). In addition, with respect to 100 pbw cyclohexanone, the amount of the oximation catalyst may be 10-50 pbw, preferably 25-40 pbw.

There is no particular restriction on the specific form of the titanium silicalite molecular sieve catalyst in the present invention; namely, the titanium silicalite molecular sieve catalyst may be a non-preformed titanium silicalite molecular sieve catalyst or a preformed titanium silicalite molecular sieve catalyst. For the convenience of separation between the titanium silicalite molecular sieve catalyst and the ammoximation reaction product, the titanium silicalite molecular sieve catalyst preferably is a preformed titanium silicalite molecular sieve catalyst. The preformed titanium silicalite molecular sieve catalyst usually consists of titanium silicalite molecular sieves and a support, wherein, based on the total weight of the preformed titanium silicalite molecular sieve catalyst, the content of the support may be 40-90 wt%, preferably 50-80 wt%; the content of the titanium silicalite molecular sieves may be 10-60 wt%, preferably 20-50 wt%.

There is no particular requirement for the support in the titanium silicalite molecular sieve catalyst; namely, the support may be any common support for preformed catalysts in the art. For example, the support may be a porous heat-resistant inorganic oxide and/or silicate. Specifically, for example, the support may be at least one of aluminum oxide, silicon oxide, titanium oxide, magnesium oxide, zirconium oxide, thorium oxide, beryllium oxide and clay, preferably at least one of aluminum oxide, silicon oxide, magnesium oxide, and zirconium oxide.

In addition, the titanium silicalite molecular sieve catalyst may be obtained commercially, or prepared with any method that is well known to those skilled in the art. For example, the titanium silicalite molecular sieve catalyst TS-1 may be prepared with the method disclosed in 'Cyclohexane Oxidation Catalyzed by Titanium Silicalite (TS-1) with Hydrogen Peroxide, Journal of Natural Gas Chemistry 2001, 10(4):295-307', or prepared with the method disclosed in CN101279959A, and will not be detailed further here.

In view that the ammoximation reaction system provided in the present invention is nonhomogeneous system, preferably the ammoximation reaction is executed under a stirring condition, in order to make the raw materials of reaction and the oximation catalyst contact and sufficient mass transfer. The stirring is carried out in a way that the entire reaction system is mixed homogeneously and the reactants can achieve sufficient mass transfer to each other. That is well known to those skilled in the art, and will not be detailed further here. Moreover, to make the raw materials of reaction (cyclohexanone, hydrogen peroxide, and ammonia, etc.) contact fully to achieve mass transfer, preferably the materials are charged by continuous charging through forced external circulation in the ammoximation reaction. Wherein, the circulation capacity shall be determined on a basis that the materials can fully contact for achieving mass transfer and the discharging capacity is appropriate.

There is no particular restriction on the kind and amount of the inert organic solvent in the present invention, as long as the cyclohexanone oxime in the ammoximation reaction product is dissolved essentially in the inert organic solvent to form an extract phase and the oximation catalyst and water are essentially insoluble in the inert organic solvent but form a raffinate phase. For example, the inert organic solvent may be selected from at least one of C5-C10 alkane, C5-C10 haloalkane, C6-C8 aromatic hydrocarbon, and C6-C8 halogenated aromatic hydrocarbon. The alkane and haloalkane may be of linear chain type, branched chain type, or ring type. Specifically, examples of the C5-C10 alkane include at least one *n*-hexane, cyclohexane, cyclopentane, and methyl cyclohexane. Examples of the C5-C10 haloalkane include at least one of chlorohexane, chlorocyclohexane, dichlorohexane, chloropentane, and bromopentane. Examples of the C6-C8 aromatic hydrocarbon include at least one of benzene, methyl benzene, dimethyl benzene, and ethyl benzene. Examples of the C6-C8 halogenated aromatic hydrocarbon include at least one of chlorobenzene, 2-chlorotoluene, and 3-chloroethylbenzene. Moreover, with respect to 1 mol cyclohexanone, the amount of the inert organic solvent preferably is 2-4 mol.

The caprolactam preparation method provided in the present invention comprises: separating the oximation catalyst from the raffinate phase, and reusing the oximation catalyst in the ammoximation reaction; thus, the oximation catalyst can be recovered and reused, and thereby the production cost can be reduced further. It should be noted that the oximation catalyst separated from the extract phase and returned to the ammoximation reaction may contain some water, i.e., the oximation catalyst may be directly returned in water phase to the ammoximation reaction, and the water content shall be appropriate to maintain water balance in the ammoximation reaction system. That is well known to those skilled in the art, and will not be detailed further here. Moreover, the method for separating the oximation catalyst from the raffinate phase usually is a solid-liquid separation method; specifically, the separation may be carried out in a decanter or a membrane filter.

There is no particular restriction on the amount of oleum in the Beckmann rearrangement reaction process. For example, with respect to 1 mol cyclohexanone, the amount of the oleum may be 0.8-1.6 mol, preferably 1.0-1.5 mol. The oleum refers to sulfuric acid solution of sulfuric anhydride, which is well known to those skilled in the art. The process for preparing caprolactam sulfonate through a Beckmann rearrangement reaction between cyclohexanone oxime and oleum is well known to those skilled in the art. Specifically, the reaction may be as follows:

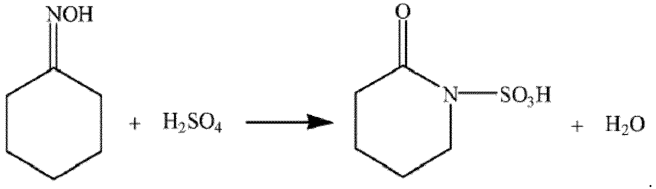

The conditions of the Beckmann rearrangement reaction may be selected conventionally in the art, and usually include a reaction temperature and a reaction time; specifically, the reaction temperature may be 80-95 °C, and the reaction time may be 0.1-2 h, for example.

According to the caprolactam preparation method provided in the present invention, to ensure that the cyclohexanone oxime is converted into caprolactam sulfonate almost completely, the method comprises: curing the product of the Beckmann rearrangement reaction after the Beckmann rearrangement reaction and before the neutralization reaction, and then controlling the cured product to have the neutralization reaction. The curing conditions are 100-140 °C curing temperature and 0.5-3 h curing time.

Since the Beckmann rearrangement reaction is an exothermic reaction, the inert organic solvent in the Beckmann rearrangement reaction system may be gasified, condensed, and then recovered, utilizing the heat generated in the rearrangement reaction. The inert organic solvent that has not been gasified in the Beckmann rearrangement process may be further gasified, condensed, and recovered in the curing process. Moreover, the condensed inert organic solvent may be mixed with ammonia to remove the entrained acid, and then the inert organic solvent may be reused.

There is no particular restriction on the amount of the ammonia used in the neutralization reaction process in the present invention, as long as the caprolactam sulfonate obtained through the Beckmann rearrangement reaction can be converted into caprolactam almost completely. Usually, with respect to 1 mol cyclohexanone, the amount of the ammonia used in the neutralization reaction may be 1-8 mol, preferably 2-5 mol.

There is no particular restriction on the conditions of the neutralization reaction. Usually, the conditions of the neutralization reaction include reaction temperature, reaction pressure, pH, and reaction time. To facilitate the neutralization reaction, the reaction temperature preferably is 45-65 °C, the absolute pressure of reaction preferably is 16-20 kPa, and the value of pH preferably is 4.5-6.5. Moreover, though longer reaction time is helpful for improving the conversion ratio of the reactants and the yield of the reaction product, the conversion ratio of the reactants and the yield of the reaction product will not be improved further apparently if the reaction time is excessively long. In comprehensive consideration, including effect and efficiency, etc., the reaction time preferably is 2-10 h. After the reaction is finished, the caprolactam, which is a light phase, can be separated from the ammonium sulfate mother liquid, which is a heavy phase, and then can be fed into the follow-up refinement procedure to produce a caprolactam finished product; the ammonium sulfate mother liquid is continuously evaporated and concentrated under the action of the heat generated in the neutralization reaction under the neutralization reaction conditions; thus, the ammonium sulfate precipitates in a form of ammonium sulfate crystals, and then is treated subsequently by centrifugation and dehydration, to obtain an ammonium sulfate finished product, which is packed and sold out.

Hereunder the present invention will be detailed in embodiments.

In the following embodiments and reference examples, the oximation catalyst is a titanium silicalite molecular sieve catalyst (TS-1), which is prepared with the method described in the lines 9-24 on page 296 of the document [Cyclohexane Oxidation Catalyzed by Titanium Silicalite (TS-1) with Hydrogen Peroxide, Journal of Natural Gas Chemistry 2001, 10(4), 295-307], wherein, the content of titanium oxide is 2.5wt%.

In the following embodiments and reference examples, the yield of caprolactam and the yield of ammonium sulfate are calculated with the following formulae: yield of caprolactam = (concentration of caprolactam in the light phase x weight of the light phase) / theoretical yield of caprolactam x 100 %, yield of ammonium sulfate = (purity of ammonium sulfate in the heavy phase x weight of the heavy phase) / theoretical yield of ammonium sulfate x 100 %.

### Embodiment 1

This embodiment is provided to describe the caprolactam preparation method provided in the present invention.

100 t water solution of oximation catalyst is loaded into an ammoximation reactor, wherein, the content of the oximation catalyst is 3 wt%, then ammonia gas, cyclohexanone, and hydrogen peroxide solution (at 27.5 wt% concentration) are charged into the ammoximation reactor at a flow rate of 1.94 t/h, 10.9 t/h and 15.2 t/h, respectively, for ammoximation reaction, the reaction temperature is controlled at 90-100 °C, the reaction pressure is 300 kPa (absolute pressure), and the materials are held in the ammoximation reactor for 70 min; thus, a water solution of cyclohexanone oxime is obtained. Cyclohexane fed at 33 t/h flow rate is mixed intensively with the water solution of cyclohexanone oxime from the ammoximation reactor at 88 t/h flow rate (with respect to 1 mol cyclohexanone, the amount of cyclohexane is 3 mol), and then the mixture is charged into a hydrocyclone separator. The heavy phase in the hydrocyclone separator is the water solution of oximation catalyst, and water is filtered via a membrane filter at 60 t/h flow rate, and then is returned to the ammoximation reaction system and reused. The light phase in the hydrocyclone separator is the cyclohexanone oxime solution; after settlement and removal of some water content, the cyclohexanone oxime solution is charged into a Beckmann rearrangement reactor, in which the cyclohexanone oxime is mixed with oleum at 15.5 t/h flow rate (the content of free SO₃ is 20 wt%) at a molar ratio of 1:1.5; then, the reaction temperature in the Beckmann rearrangement reactor is controlled at 86-90 °C and held for 1 h for reaction; thus, a rearrangement liquid is obtained. The rearrangement liquid is charged at 27.7 t/h flow rate into a curing reactor for curing reaction, the curing reaction temperature is controlled at 120-130 °C, and the curing time is controlled to be 1.5 h. The cured rearrangement liquid is introduced at 27.7 t/h flow rate into an ammonium sulfate crystallizer, and ammonia gas is introduced at 7 t/h flow rate into the ammonium sulfate crystallizer at the same time (with respect to 1 mol cyclohexanone, the amount of the ammonia gas is 3.7 mol) for neutralization reaction, the temperature in the ammonium sulfate crystallizer is controlled at 45 °C, the absolute pressure is controlled at 16 kPa, and the value of pH is controlled at 4.5. After reaction for 8 h, the reaction product is treated by settling separation, to obtain a light phase that contains caprolactam and a heavy phase that contains ammonium sulfate. Through calculation, it is ascertained that the conversion ratio of cyclohexanone is 99.99%, the selectivity for cyclohexanone oxime is 99.32%, the yield of caprolactam is 99.88%, and the yield of ammonium sulfate is 99.81%. The gas phase in the Beckmann rearrangement reactor and the curing reactor is condensed and then the condensate is fed into an ammonia washing tank at 33 t/h flow rate, and is mixed with ammonia at 10% concentration fed at 7 t/h flow rate to wash off the entrained acid; next, the heavy phase is fed into the ammonium sulfate crystallizer, while the cyclohexane is returned to the previous procedure and reused.

### Embodiment 2

This embodiment is provided to describe the caprolactam preparation method provided in the present invention.

100 t water solution of oximation catalyst is loaded into an ammoximation reactor, wherein, the content of the oximation catalyst is 3 wt%, then ammonia gas, cyclohexanone, and hydrogen peroxide solution (at 27.5 wt% concentration) are charged into the ammoximation reactor at a flow rate of 5.19 t/h, 27.22 t/h and 38.46 t/h respectively for ammoximation reaction, the reaction temperature is controlled at 80-85 °C, the reaction pressure is 250 kPa (absolute pressure), and the materials are held in the ammoximation reactor for 80 min; thus, a water solution of cyclohexanone oxime is obtained. Cyclohexane fed at 82 t/h flow rate is mixed intensively with the water solution of cyclohexanone oxime from the ammoximation reactor at 136 t/h flow rate (with respect to 1mol cyclohexanone, the amount of cyclohexane is 3 mol), and then the mixture is charged into a hydrocyclone separator. The heavy phase in the hydrocyclone separator is the water solution of oximation catalyst, and water is filtered via a membrane filter at 65 t/h flow rate, and then is returned to the ammoximation reaction system and reused. The light phase in the hydrocyclone separator is the cyclohexanone oxime solution; after settlement and removal of some water content, the cyclohexanone oxime solution is charged into a Beckmann rearrangement reactor, in which the cyclohexanone oxime is mixed with oleum at 22 t/h flow rate (the content of free SO₃ is 20 wt%, the same below) at a molar ratio of 1:0.8; then, the reaction temperature in the Beckmann rearrangement reactor is controlled at 86-90 °C and held for 1 h for reaction; thus, a rearrangement liquid is obtained. The rearrangement liquid is charged at 53 t/h flow rate into a curing reactor for curing reaction, the curing reaction temperature is controlled at 120-130 °C, and the curing time is controlled to be 1.5 h. The cured rearrangement liquid is introduced at 53 t/h flow rate into an ammonium sulfate crystallizer, and ammonia gas is introduced at 23.6 t/h flow rate into the ammonium sulfate crystallizer at the same time (with respect to 1mol cyclohexanone, the amount of the ammonia gas is 5 mol) for neutralization reaction, the temperature in the ammonium sulfate crystallizer is controlled at 65 °C, the absolute pressure is controlled at 20 kPa, and the value of pH is controlled at 5.5. After reaction for 4 h, the reaction product is treated by settling separation, to obtain a light phase that contains caprolactam and a heavy phase that contains ammonium sulfate. Through calculation, it is ascertained that the conversion ratio of cyclohexanone is 99.97 %, the selectivity for cyclohexanone oxime is 99.33 %, the yield of caprolactam is 99.68 %, and the yield of ammonium sulfate is 98.89 %. The gas phase in the Beckmann rearrangement reactor and the curing reactor is condensed and then the condensate is fed into an ammonia washing tank at 33 t/h flow rate, and is mixed with ammonia at 10 % concentration fed at 26.3 t/h flow rate to wash off the entrained acid; next, the heavy phase is fed into the ammonium sulfate crystallizer, while the cyclohexane is returned to the previous procedure and reused.

### Embodiment 3

This embodiment is provided to describe the caprolactam preparation method provided in the present invention.

100 t water solution of oximation catalyst is loaded into an ammoximation reactor, wherein, the content of the oximation catalyst is 3 wt%, then ammonia gas, cyclohexanone, and hydrogen peroxide solution (at 27.5 wt% concentration) are charged into the ammoximation reactor at a flow rate of 2.45 t/h, 13.6 t/h and 18.5 t/h respectively for ammoximation reaction, the reaction temperature is controlled at 85-90 °C, the reaction pressure is 200 kPa (absolute pressure), and the materials are held in the ammoximation reactor for 90 min; thus, a water solution of cyclohexanone oxime is obtained. Cyclohexane fed at 54.4 t/h flow rate is mixed intensively with the water solution of cyclohexanone oxime from the ammoximation reactor at 91 t/h flow rate (with respect to 1mol cyclohexanone, the amount of cyclohexane is 4 mol), and then the mixture is charged into a hydrocyclone separator. The heavy phase in the hydrocyclone separator is the water solution of oximation catalyst, and water is filtered via a membrane filter at 60 t/h flow rate, and then is returned to the ammoximation reaction system and reused. The light phase in the hydrocyclone separator is the cyclohexanone oxime solution; after settlement and removal of some water content, the cyclohexanone oxime solution is charged into a Beckmann rearrangement reactor, in which the cyclohexanone oxime is mixed with oleum at 15.8 t/h flow rate at a molar ratio of 1:1.2; then, the reaction temperature in the Beckmann rearrangement reactor is controlled at 86-90 °C and held for 2 h for reaction; thus, a rearrangement liquid is obtained. The rearrangement liquid is charged at 32 t/h flow rate into a curing reactor for curing reaction, the curing reaction temperature is controlled at 120-130 °C, and the curing time is controlled to be 1.5 h. The cured rearrangement liquid is introduced at 32 t/h flow rate into an ammonium sulfate crystallizer, and ammonia gas is introduced at 7 t/h flow rate into the ammonium sulfate crystallizer at the same time (with respect to 1mol cyclohexanone, the amount of the ammonia gas is 3mol) for neutralization reaction, the temperature in the ammonium sulfate crystallizer is controlled at 50 °C, the absolute pressure is controlled at 18 kPa, and the value of pH is controlled at 5. After reaction for 2 h, the reaction product is treated by settling separation, to obtain a light phase that contains caprolactam and a heavy phase that contains ammonium sulfate. Through calculation, it is ascertained that the conversion ratio of cyclohexanone is 99.95 %, the selectivity for cyclohexanone oxime is 99.31 %, the yield of caprolactam is 99.52 %, and the yield of ammonium sulfate is 98.95 %. The gas phase in the Beckmann rearrangement reactor and the curing reactor is condensed and then the condensate is fed into an ammonia washing tank at 33 t/h flow rate, and is mixed with ammonia at 10 % concentration fed at 7 t/h flow rate to wash off the entrained acid; next, the heavy phase is fed into the ammonium sulfate crystallizer, while the cyclohexane is returned to the previous procedure and reused.

### Embodiment 4

This embodiment is provided to describe the caprolactam preparation method provided in the present invention.

Caprolactam is prepared with the method used in the embodiment 1, but the cyclohexane inert organic solvent is replaced with methyl benzene inert organic solvent in the same amount at the same flow rate; thus, a light phase that contains caprolactam and a heavy phase that contains ammonium sulfate are obtained. Through calculation, it is ascertained that the conversion ratio of cyclohexanone is 99.89 %, the selectivity for cyclohexanone oxime is 99.34 %, the yield of caprolactam is 99.12 %, and the yield of ammonium sulfate is 98.32 %.

### Reference example 1

This reference example is provided to describe the reference caprolactam preparation method.

Caprolactam is prepared with the method used in the embodiment 2, but the 100 t water solution of oximation catalyst (the content of the oximation catalyst is 3 wt%) is replaced with 100 t cyclohexane solution of oximation catalyst (the content of the oximation catalyst is 3 wt%). Through calculation, it is ascertained that the conversion ratio of cyclohexanone is 98.32 %, the selectivity for cyclohexanone oxime is 98.87 %, the yield of caprolactam is 97.75 %, and the yield of ammonium sulfate is 98.86 %.

### Reference example 2

This reference example is provided to describe the reference caprolactam preparation method.

Caprolactam is prepared with the method used in the embodiment 2, but the temperature of the ammoximation reaction system is controlled at 40-45 °C. Through calculation, it is ascertained that the conversion ratio of cyclohexanone is 94.55 %, the selectivity for cyclohexanone oxime is 98.65 %, the yield of caprolactam is 98.66 %, and the yield of ammonium sulfate is 98.81 %.

It is seen from the above results: when caprolactam is prepared with the method provided in the present invention, higher cyclohexanone conversion ratio, higher selectivity for cyclohexanone oxime, and higher caprolactam yield can be attained. Furthermore, with the method provided in the present invention, the refinement procedures, such as water scrubbing and rectification, in the cyclohexanone oxime preparation process can be omitted, the process flow and production cycle can be greatly shortened; moreover, the inert organic solvent can be recovered utilizing the heat generated in the rearrangement reaction and then reused. Thus, the annual yield is improved on the basis of the existing installation and the production cost is reduced. With the method, great social and economic benefits can be obtained.

## Claims

1. A caprolactam preparation method, comprise the following steps:
(1) Controlling cyclohexanone, ammonia, and hydrogen peroxide to have an ammoximation reaction in an aqueous solvent at 80-100 °C temperature in the presence of an oximation catalyst, without any organic solvent, to obtain a solution that contains cyclohexanone oxime;
(2) Extracting the obtained solution that contains cyclohexanone oxime in step (1) with an inert organic solvent, to obtain an extract phase that contains cyclohexanone oxime and a raffinate phase that contains the oximation catalyst and water;
(3) Controlling the extract phase to have a Beckmann rearrangement reaction with oleum, and controlling the product of the Beckmann rearrangement reaction to have a neutralization reaction with ammonia,
wherein step (2) further comprises: separating the oximation catalyst from the raffinate phase, and reusing the oximation catalyst in the ammoximation reaction, and
wherein step (3) further comprises: curing the product of the Beckmann rearrangement reaction after the Beckmann rearrangement reaction is finished and before the neutralization reaction is started, and then controlling the cured product to have the neutralization reaction; wherein the curing conditions are 100-140 °C curing temperature and 0.5-3 h curing time.

2. The method according to claim 1, wherein in step (1), the aqueous solvent is water, and, with respect to 1 mol cyclohexanone, the amount of water in the ammoximation reaction system is 6-500 mol, preferably 20-50 mol.

3. The method according to claim 1 or 2, wherein the pressure of the ammoximation reaction is 50-300 kPa and wherein the reaction time is 10-300 min, preferably wherein the pressure of the ammoximation reaction is 200-300 kPa and the reaction time is 50-100 min.

4. The method according to any of claims 1-3, wherein the hydrogen peroxide is added in a form of hydrogen peroxide solution.

5. The method according to any of claims 1-4, wherein in step (1), with respect to 1 mol cyclohexanone, the amount of the hydrogen peroxide is 1-1.5 mol, and the amount of the ammonia is 1-1.5 mol.

6. The method according to any of claims 1-5, wherein, the oximation catalyst is a titanium silicalite molecular sieve catalyst.

7. The method according to any of claims 1-6, wherein with respect to 1 mol cyclohexanone, the amount of the inert organic solvent used in step (2) is 2-4 mol, wherein the inert organic solvent is preferably selected from at least one of C5-C10 alkane, C5-C10 haloalkane, C6-C8 aromatic hydrocarbon, and C6-C8 halogenated aromatic hydrocarbon.

8. The method according to any of claims 1-7, wherein with respect to 1 mol cyclohexanone, the amount of the oleum used in the step (3) is 0.8-1.6 mol.

9. The method according to any of claims 1-8, wherein the conditions of the Beckmann rearrangement reaction are: 80-95 °C reaction temperature and 0.1-2 h reaction time.

10. The method according to any of claims 1-9, wherein in step (1), with respect to 1 mol cyclohexanone, the amount of the ammonia used in the neutralization reaction is 1-8 mol.

11. The method according to any of claims 1-10, wherein the conditions of the neutralization reaction are 45-65 °C reaction temperature, 16-20 kPa reaction pressure, pH=4.5-6.5 and 2-10 h reaction time.

## Patentansprüche

1. Verfahren zur Herstellung von Caprolactam, das die folgenden Schritte umfasst:
(1) Regulierung von Cyclohexanon, Ammoniak und Wasserstoffperoxid, um eine Ammoximierungsreaktion in einem wässrigen Lösungsmittel bei einer Temperatur von 80-100 °C in der Gegenwart eines Oximierungskatalysators ohne jegliches organisches Lösungsmittel durchzuführen, um eine Lösung zu erhalten, die Cyclohexanonoxim enthält,
(2) Extrahieren der erhaltenen Lösung, die Cyclohexanonoxim enthält, aus Schritt (1) mit einem inerten organischen Lösungsmittel, um eine Extraktphase, die Cyclohexanonoxim enthält, und eine Raffinatphase, die den Oximierungskatalysator und Wasser enthält, zu erhalten,
(3) Regulierung der Extraktphase, um eine Beckmann-Umlagerungsreaktion mit Oleum durchzuführen, und Regulierung des Produkts der Beckmann-Umlagerungsreaktion, um eine Neutralisationsreaktion mit Ammoniak durchzuführen,
wobei Schritt (2) ferner umfasst, dass der Oximierungskatalysator von der Raffinatphase abgetrennt wird und der Oximierungskatalysator in der Ammoximierungsreaktion wiederverwendet wird und
wobei Schritt (3) weiterhin umfasst, dass das Produkt der Beckmann Umlagerungsreaktion gehärtet wird, nachdem die Beckmann-Umlagerungsreaktion beendet ist und bevor die Neutralisationsreaktion gestartet wird, und dann das gehärtete Produkt reguliert wird, um die Neutralisationsreaktion durchzuführen, wobei die Härtungsbedingungen 100-140 °C Härtungstemperatur und 0,5-3 h Härtungszeit betragen.

2. Verfahren nach Anspruch 1, bei dem in Schritt (1) das wässrige Lösungsmittel Wasser ist, und, bezogen auf 1 mol Cyclohexanon, die Menge an Wasser im Reaktionssystem der Ammoximierung 6-500 mol, vorzugsweise 20-50 mol beträgt.

3. Verfahren nach Anspruch 1 oder 2, bei dem der Druck der Ammoximierungsreaktion 50-300 kPa beträgt und bei dem die Reaktionszeit 10-300 min beträgt, wobei vorzugsweise der Druck der Ammoximierungsreaktion 200-300 kPa und die Reaktionszeit 50-100 min beträgt.

4. Verfahren nach einem der Ansprüche 1 bis 3, bei dem das Wasserstoffperoxid in Form einer Wasserstoffperoxidlösung zugegeben wird.

5. Verfahren nach einem der Ansprüche 1 bis 4, bei dem in Schritt (1), bezogen auf 1 Mol Cyclohexanon, die Menge des Wasserstoffperoxids 1-1,5 Mol und die Menge des Ammoniaks 1-1,5 mol beträgt.

6. Verfahren nach einem der Ansprüche 1 bis 5, bei dem der Oximierungskatalysator ein Titansilikalit-Molekularsieb-Katalysator ist.

7. Verfahren nach einem der Ansprüche 1 bis 6, bei dem, bezogen auf 1 mol Cyclohexanon, die Menge des in Schritt (2) verwendeten inerten organischen Lösungsmittels 2-4 mol beträgt, wobei das inerte organische Lösungsmittel vorzugsweise ausgewählt ist aus mindestens einem von C5-C10-Alkan, C5-C10-Halogenalkan, aromatischem C6-C8-Kohlenwasserstoff und halogeniertem aromatischem C6-C8-Kohlenwasserstoff.

8. Verfahren nach einem der Ansprüche 1 bis 7, bei dem, bezogen auf 1 mol Cyclohexanon, die Menge des in Schritt (3) verwendeten Oleums 0,8-1,6 mol beträgt.

9. Verfahren nach einem der Ansprüche 1 bis 8, bei dem die Bedingungen der Beckmann-Umlagerungsreaktion 80-95 °C Reaktionstemperatur und 0,1-2 h Reaktionszeit betragen.

10. Verfahren nach einem der Ansprüche 1 bis 9, bei dem in Schritt (1), bezogen auf 1 mol Cyclohexanon, die Menge des in der Neutralisationsreaktion verwendeten Ammoniaks 1-8 mol beträgt.

11. Verfahren nach einem der Ansprüche 1 bis 10, bei dem die Bedingungen der Neutralisationsreaktion 45-65 °C Reaktionstemperatur, 16-20 kPa Reaktionsdruck, pH = 4,5-6,5 und 2-10 h Reaktionszeit betragen.

## Revendications

1. Procédé de préparation de caprolactame, comprenant les étapes suivantes :
1) faire réagir de la cyclohexanone, de l'ammoniac et du peroxyde d'hydrogène dans une réaction d'ammoximation dans un solvant aqueux à une température de 80-100°C en présence d'un catalyseur d'oximation, sans aucun solvant organique, pour obtenir une solution qui contient de la cyclohexanone oxime ;
2) extraire la solution obtenue qui contient de la cyclohexanone oxime dans l'étape (1) par un solvant organique inerte, pour obtenir une phase d'extrait qui contient de la cyclohexanone oxime et une phase de raffinat qui contient le catalyseur d'oxymation et de l'eau ;
3) faire réagir la phase d'extrait dans une réaction de réarrangement de Beckmann avec de l'oléum, et faire réagir le produit de la réaction de réarrangement de Beckmann dans une réaction de neutralisation avec de l'ammoniac,
dans lequel l'étape (2) comprend en outre : la séparation du catalyseur d'oximation à partir de la phase de raffinat, et la réutilisation du catalyseur d'oximation dans la réaction d'ammoximation, et
dans lequel l'étape (3) comprend en outre : le durcissement du produit de la réaction de réarrangement de Beckmann après que la réaction de réarrangement de Beckmann soit terminée et avant que la réaction de neutralisation ne soit commencée, puis l'opération consistant à faire réagir le produit durci dans la réaction de neutralisation, les conditions de durcissement étant une température de durcissement de 100 - 140°C et un temps de durcissement de 0,5 - 3h.

2. Procédé selon la revendication 1, dans lequel, dans l'étape (1), le solvant aqueux est l'eau, et, par rapport à 1 mole de cyclohexanone, la quantité d'eau dans le système de réaction d'ammoximation est de 6 - 500 moles, de préférence de 20 - 50 moles.

3. Procédé selon l'une des revendications 1 ou 2, dans lequel la pression de la réaction d'ammoximation est de 50 - 300kPa, et dans lequel le temps de réaction est de 10 - 300 min, de préférence dans lequel la pression de la réaction d'ammoximation est de 200 - 300 kPa, et le temps de réaction est de 50 - 100 min.

4. Procédé selon l'une quelconque des revendications 1 à 3, dans lequel le peroxyde d'hydrogène est ajouté sous une forme de solution de peroxyde d'hydrogène.

5. Procédé selon l'une quelconque des revendications 1 à 4, dans lequel, à l'étape (1), par rapport à 1 mole de cyclohexanone, la quantité du peroxyde d'hydrogène est de 1-1,5 mole, et la quantité de l'ammoniac est de 1 - 1,5 mole.

6. Procédé selon l'une quelconque des revendications 1 à 5, dans lequel le catalyseur d'oxydation est un catalyseur tamis moléculaire de silicalite de titane.

7. Procédé selon l'une quelconque des revendications 1 à 6, dans lequel, par rapport à 1 mole de cyclohexanone, la quantité de solvant organique inerte utilisée à l'étape (2) est de 2 - 4 moles ; dans lequel le solvant organique inerte est, de préférence, choisi parmi au moins l'un d'un alcane en C5-C10, un haloalcane en C5-C10, un hydrocarbure aromatique en C6-C8 et un hydrocarbure aromatique halogéné en C6-C8.

8. Procédé selon l'une quelconque des revendications 1 à 7, dans lequel, par rapport à 1 mole de cyclohexanone, la quantité de l'oléum utilisée dans l'étape (3) est de 0,8 - 1,6 mole.

9. Procédé selon l'une quelconque des revendications 1 à 8, dans lequel les conditions de la réaction de réarrangement de Beckmann sont : température de réaction de 80 - 95°C et temps de réaction de 0,1- 2h.

10. Procédé selon l'une quelconque des revendications 1 à 9, dans lequel, à l'étape (1), par rapport à 1 mole de cyclohexanone, la quantité d'ammoniac utilisée dans la réaction de neutralisation est de 1 - 8 moles.

11. Procédé selon l'une quelconque des revendications 1 à 10, dans lequel les conditions de la réaction de neutralisation sont une température de réaction de 45 - 65°C, une pression de réaction de 16 - 20 kPa, un pH = 4,5 - 6,5 et un temps de réaction de 2 - 10h.
